Europäisches Patentamt

European Patent Office

Office européen des brevets

(19)

(11) **EP 1 056 440 B1**

(12) **FASCICULE DE BREVET EUROPEEN**

(45) Date de publication et mention
de la délivrance du brevet:
**15.09.2004 Bulletin 2004/38**

(21) Numéro de dépôt: **99904922.4**

(22) Date de dépôt: **22.02.1999**

(51) Int Cl.⁷: **A61K 7/48**

(86) Numéro de dépôt international:
**PCT/FR1999/000389**

(87) Numéro de publication internationale:
**WO 1999/042082 (26.08.1999 Gazette 1999/34)**

(54) **COMPOSITION COSMETIQUE COMPRENANT DES OLIGO-ELEMENTS PROVENANT D'EAUX MINERALES ET CHELATES PAR DES HYDROLYSATS DE PROTEINES VEGETALES**

KOSMETISCHES MITTEL, ENTHALTEND CHELATE AUS PFLANZLICHEN PROTEINHYDROLYSATEN UND SPURENELEMENTEN AUS MINERALWÄSSERN

COSMETIC COMPOSITION COMPRISING TRACE ELEMENTS DERIVED FROM MINERAL WATERS AND CHELATED BY PLANT PROTEIN HYDROLYSATES

(84) Etats contractants désignés:
**AT BE CH CY DE DK ES FI FR GB GR IE IT LI LU MC NL PT SE**

(30) Priorité: **23.02.1998 FR 9802130**

(43) Date de publication de la demande:
**06.12.2000 Bulletin 2000/49**

(73) Titulaire: **Pierre Fabre Dermo-Cosmetique**
**92100 Boulogne-Billancourt (FR)**

(72) Inventeurs:
- **FORT LACOSTE, Lydie**
  **F-31120 Roquettes (FR)**
- **PEYROT, Nicole**
  **F-31400 Toulouse (FR)**

- **NAVARRO, Roger**
  **F-09100 Pamiers (FR)**
- **TOURNAY, Alain**
  **F-63830 Durtol (FR)**

(74) Mandataire: **Ahner, Francis et al**
**Cabinet Régimbeau**
**20, rue de Chazelles**
**75847 Paris cedex 17 (FR)**

(56) Documents cités:
**WO-A-87/05503          WO-A-95/13793**
**FR-A- 2 096 845        FR-A- 2 539 005**
**FR-A- 2 694 692        FR-A- 2 727 862**
**GB-A- 1 575 577**

**Description**

La présente invention concerne notamment une composition cosmétique pour améliorer l'état et l'aspect de la peau et des phanères, pour stimuler la régénérescence cellulaire et prévenir contre le vieillissement cutané. Cette composition comprend comme principe actif des oligo-éléments provenant d'eaux minérales et des hydrolysats de protéines végétales qui chélatent lesdits oligo-éléments. La présente invention porte aussi sur le procédé de préparation de cette composition.

Il est bien connu que les oligo-éléments (notamment Al, Ag, Ca, Cl, Cr, Co, Cu, Fe, F, Ge, Li, Mg, Mn, Na, Ni, Au, P, K, Se, S, Sr, Zn) sont essentiels pour de nombreuses fonctions physiologiques et pour diverses réactions biochimiques. Par exemple, on les retrouve impliqués dans de nombreuses réactions enzymatiques, soit associés directement aux sites actifs des enzymes, soit en participant à la catalyse sous forme libre. Ainsi, dans la majorité des cascades biochimiques (voies du métabolisme, du catabolisme, et régulatrices), on retrouve une forte présence d'oligo-éléments illustrant ainsi leur rôle primordial dès les origines de la vie. En particulier, la synthèse d'ADN, d'ARN, l'activité des ribosomes, le transfert des ARNt, et la modulation de l'expression des gènes par protéines régulatrices, ne pourrait se faire en l'absence desdits oligo-éléments.

Un autre processus biochimique bien connu et vital pour un organisme vivant, est celui de la respiration. La cascade de transferts d'électrons, dans les mitochondries, met en oeuvre de multiples enzymes dépendant d'oligo-éléments. La chaîne d'oxydo-réduction ne pourrait fonctionner sans ces oligo-éléments. De plus, la teneur particulièrement riche et variée en oligo-éléments dans les media de culture cellulaire démontre leur importance : Il n'est pas rare en effet de constater la mort prématurée d'une culture si un seul élément vient à manquer.

Le processus de détoxification des cellules implique aussi la présence d'oligo-éléments, en particulier pour tout ce qui concerne l'élimination des radicaux libres dans la cellule. La sénescence cellulaire est étroitement lié à l'accumulation de mutations dans le génome notamment due à l'accumulation des radicaux libres.

On comprend donc bien le rôle essentiel des oligo-éléments sur la croissance cellulaire, sur la détoxification contre les stress oxydants dus aux agressions internes et externes, sur l'intégrité fonctionnelle des gènes et plus macroscopiquement, sur le maintien des qualités mécaniques de la peau. Les mécanismes d'action au niveau cutané de certains oligo-éléments ont été étudiés notamment pour le zinc et pour le sélénium, Dreno, 1995, et Leccia et al., 1993.

EP 0 699 432 décrit un moyen d'apporter des oligo-éléments à la peau par l'utilisation d'eaux minérales naturelles, notamment des eaux thermales. Elles sont judicieusement introduites dans des préparations thermo-cosmétiques, notamment pour améliorer la tolérance de certains principes actifs. Lorsqu'on évoque l'action des eaux thermales on pense en général à la balance entre les cations et les anions les plus abondants. Cependant, le mode d'action de ces eaux a été revu en tenant compte plus particulièrement du contenu total en oligo-éléments et plus précisément en considérant des éléments susceptibles d'action pharmacologique, Simonoff, 1988.

Des agents chélateurs ont été employés en dermo-cosmétique, notamment pour piéger le fer et le cuivre qui sont les métaux les plus fréquemment impliqués dans la catalyse des réactions radicalaires se produisant in vivo, Deby et al., 1993. Par exemple, l'acide phytique est employé comme agent chélatant dans WO 96/37187, mais il comporte un spectre de chélation très étroit, ce qui limite son utilisation. Dans un autre brevet (FR 2 706 301), on trouve des chélateurs de métaux dans un produit de nuit anti-âge.

L'absorption des oligo-éléments et le passage à l'intérieur des couches cutanées ne peuvent être réalisés efficacement qu'en utilisant des vecteurs.

Des vecteurs ont été décrits dans l'art antérieur. FR 2 694 692 concerne des vecteurs à base d'acides gras tels que des lipides riches en acides linoléiques ou linoléniques, ou bien des acides aminées dermo-conductibles ainsi que des chélateurs à base d'acides aminés. La réelle efficacité du transport dépend à la fois du spectre de chélation des acides aminés employés et de leurs capacités à traverser l'épiderme, ce qui limite leurs utilisations in fine. WO 84/04885 décrit l'utilisation du sang provenant d'animaux ou de constituants du sang comme vecteur d'oligo-éléments. Connaissant aujourd'hui le risque au niveau de la santé publique d'utiliser du sang provenant d'animaux (contamination par prions, virus et bactéries), sachant que les protéines chélatrices du sang (transferrine, alpha-microglobuline, transmagine, hémoglobine) possèdent un spectre de chélation très étroit (particulièrement spécifique à Fe), et enfin, considérant que lesdites protéines du sang se dénaturent très facilement et perdent ainsi leur propriété de chélation, il n'apparaît pas raisonnable d'utiliser cette source pour vectoriser les oligo-éléments à travers l'épiderme.

WO 87/05 503 décrit un mélange d'hydrolysat d'histones végétales avec de l'eau du robinet enrichie en sels minéraux. FR 2 539 005 décrit un additif alimentaire à base de sels minéraux. GB 1 575 577, US 3,969, 540 portent sur des complexes en métaux essentiels comme additifs alimentaires.

EP 487 619 décrit un procédé d'obtention d'hydrolysats de la β-caséine par voie enzymatique et l'utilisation desdits hydrolysats pour chélater le calcium (Ca) de préférence dans le but d'améliorer son absorption dans l'intestin. La β-caséine ne saurait être utile pour transporter les oligo-éléments puisque son spectre de chélation se limite au calcium.

**EP 1 056 440 B1**

En résumé, il existe un réel besoin en innovation pour des produits cosmétiques capables de fournir efficacement aux cellules de la peau des oligo-éléments variés, et sans aucun risque pour la santé. Cette innovation est l'objet de la présente demandé de brevet tout en allant plus loin car il existe une véritable synergie d'action entre les oligo-éléments et les hydrolysats de protéines végétales. Aucun document de l'art antérieur ne décrit, ni ne suggère, la présente invention telle que définie ci-après.

Ainsi, la présente invention a pour objet une nouvelle composition cosmétique associant deux principes actifs, les hydrolysats de protéines végétales et les oligo-éléments d'eaux minérales sélectionnées parmi l'eau d'Avène et l'eau de Cauterets, interagissant l'un sur l'autre et qui possèdent des activités synergiques au niveau de la prolifération cellulaire et pour la lutte contre l'accumulation de radicaux libres dans les cellules. On choisit comme source d'oligo-éléments l'eau d'Avènes et/ou l'eau de Cauterets. Les chélates proviennent de l'hydrolyse de protéines végétales, en particulier de l'hydrolyse de protéines de blé. On obtient donc une source très variée de chélates capables de lier une multitude d'oligo-éléments qui comprennent les oligo-éléments présents en abondance dans les milieux naturels (par exemple Na, Cl, Ca, Mg, P, K, S) mais aussi les oligo-éléments plus rares et possédant des propriétés thérapeutiques (par exemple Al, Ag, Au, Cr, Co, Cu, Fe, F, Ge, Li, Mn, Ni, Se, Sr, Zn).

Des extraits de protéines végétales seuls possèdent des propriétés hydratantes de la peau. Cependant, les expériences, mises en oeuvre lors de la présente invention, ont montré de manière surprenante que les hydrolysats de protéines végétales et les oligo-éléments, lorsque mis en contact l'un avec l'autre, possèdent en outre des activités synergiques au niveau de la prolifération cellulaire , de la croissance et la division des cellules, mais aussi de manière inattendue pour lutter contre l'accumulation de radicaux libres au sein des cellules. Les radicaux libres lorsqu'ils s'accumulent dans les cellules, endommagent irréversiblement l'intégrité fonctionnelle et structurelle de l'ADN. Ces dommages sont l'une des causes principales de la dégénérescence des cellules par dérégulation ou inactivation de gènes essentiels pour le bon fonctionnement cellulaire. Le phénomène d'accumulation des radicaux libres est principalement lié à l'activité des cellules, en particulier à la respiration, mais il dépend aussi de l'environnement externe (exposition aux UV, ou à des polluants). Lorsque les cellules sont en pleine croissance, et lorsqu'elles se divisent rapidement, la consommation d'oxygène augmente sensiblement, ce qui entraîne l'accumulation de radicaux libres. De même, les rayonnements UV et certains produits chimiques polluants déclenchent des réactions radicalaires en chaîne.

La présente invention se propose donc de résoudre deux problèmes à la fois : d'une part, elle stimule la prolifération cellulaire (ce qui provoque un accroissement de la respiration et l'accumulation de radicaux libres) et d'autre part, elle lutte contre l'accumulation desdits radicaux libres en stimulant le système de détoxification des cellules. En ce sens, la double action et la synergie entre les principes actifs, confèrent à la composition de la présente invention des propriétés recherchées en cosmétique dont l'amélioration de l'état et de l'aspect de la peau, la lutte contre le vieillissement, la prévention contre la dégénérescence cellulaire, la stimulation de la régénérescence cellulaire tout en luttant contre l'accumulation des radicaux libres, la lutte contre les agressions de l'environnement extérieur et contre la sécheresse cutanée.

En option, d'autres principes actifs peuvent être rajoutés dans la présente composition. Sans se limiter à un composé particulier, on peut citer de préférence :

- les rétinoïdes dont le rétinal,
- les anti-radicalaires dont la vitamine E, et les oligomères procyanidoliques (OPC),
- les anti-âges dont l'ADN, les extraits de cyanophycées,
- les hydratants dont l'acide hyaluronique, le glycolate de guanidine, et les céramides,
- les anti-sébhorréïques dont l'extrait de Sabal,
- les vitamines dont les vitamines C et E,
- les anti-inflammatoires dont l'acide glycyrrhétinique,
- les dépigmentants dont l'extrait d'hydroquinine et l'extrait de piloselle,
- les anti-chutes des cheveux dont le minoxidil,
- les acides gras essentiels (AGE) et les huiles riches en AGE,
- les amincissants,
- les cicatrisants,
- les protecteurs solaires,
- les antifongiques,
- les antibactériens,
- les antiviraux et/ou toute combinaison de ces principes actifs.

Selon un mode avantageux de la présente invention, la concentration en chélate est comprise entre 0,0001 % et 50 % en poids par rapport au poids total de la composition. Ladite composition comporte en outre un ou des véhicules appropriés pour une utilisation en cosmétologie, notamment en dermo-cosmétologie ou se présente sous la forme nutraceutique renfermant un ou plusieurs véhicules destinés à permettre son administration par voie orale.

Ainsi, on peut choisir de préparer la composition sous une forme de crème, ou sous une forme de poudre concentrée en oligo-élément, ou sous toute forme cosmétique acceptable, ou bien encore pour la mise sous forme de gélules, de capsules, de comprimés, ou toute forme destinée à une administration par voie orale.

La présente invention concerne aussi le procédé de fabrication de la composition telle que définie ci-dessus. Ce procédé comporte une étape de mise en contact des chélates avec l'eau préalablement concentrée en oligo-éléments. L'obtention des chélates est réalisée en hydrolysant les protéines végétales par voie enzymatique et/ou par voie acide.

Grâce à cette nouvelle association synergique des deux principes actifs, la présente composition peut être utilisée pour améliorer l'état et l'aspect de la peau ou des phanères, pour stimuler la régénérescence, pour prévenir contre la dégénérescence de la peau, pour lutter contre la formation des radicaux libres et contre leur effet vieillissant sur la peau, pour lutter contre la sécheresse cutanée, et/ou pour toute autre utilisation selon qu'on ajoute les autres produits optionnels décrits précédemment. Sans limiter l'étendue de la présente invention, on peut citer comme exemples d'utilisation des compositions selon l'invention : la lutte contre les bactéries, les virus, et les champignons, la prévention et la lutte contre la chute des cheveux, la lutte contre les inflammations.

On se référera aux légendes des figures pour la suite de la description et pour les exemples.

## LEGENDES

Figure 1 : Activité des chélates sur la prolifération cellulaire (fibroblastes L929).
Les chélates renfermant les oligo-éléments (chélates pleins) sont représentés par les rectangles noirs. Les chélates sans oligo-éléments (chélates vides) sont représentés par les rectangles gris clairs.
Les concentrations utilisées sont 0,25%, 0,50% et 1%.

Figure 2 : Oxygraphie (consommation en oxygène)
Les rectangles gris clairs et noirs représentent respectivement les % d'activité des chélates vides et pleins (concentration de 0.1% et 1%)par rapport au témoin.

## Procédé d'obtention des chélates

D'une part, le procédé consiste en l'hydrolyse enzymatique de protéines végétales (on utilise à sa convenance n'importe quelle protéase, par exemple la trypsine, la chymotrypsine, et les endopeptidases). L'hydrolyse peut aussi s'effectuer par voie acide pendant environ 48h à environ 50°C. Les polypeptides obtenus ont des poids moléculaires oscillants aux alentours de 50 à 1000 DA, de préférence autour de quelque centaines de dalton. D'autre part, l'eau minérale est concentrée environ 20 fois par toute technique bien connue par l'homme du métier. Lesdits polypeptides sont ensuite séchés et mis en contact avec l'eau concentrée. Le pH de l'ensemble est neutralisé si les polypeptides ont été obtenus par hydrolyse acide. La composition est ensuite atomisée de façon à obtenir une poudre riche en oligo-élément (teneur environ égale à 10% en minéraux). Dans une dernière étape, la poudre est stérilisée par ionisation.

## Action des chélates sur la prolifération cellulaire

L'action des chélates renfermant les oligo-éléments provenant d'une eau thermale (chélates pleins) a été évaluée sur les fibroblastes L929. Les chélates vides, c'est à dire sans les minéraux de l'eau, ont été utilisés comme contrôle de la prolifération cellulaire. L'utilisation de l'eau minérale seule (V. infra, tableau 2) ne présente aucune influence sur la prolifération. Le principe de ce test est basé sur la transformation enzymatique d'un sel de tétrazolium (XTT)en un produit coloré, le formazan. XXT est réduit par la déshydrogénase mitochondriale des cellules vivantes, en présence d'un agent couplant d'électrons (le coenzyme Q), en un composé hydrosoluble jaune/orangé, le formazan. On dose la quantité de formazan produit par spectrométrie à 450 nm. Les cellules sont ensemencées avec du SNN (sérum de veau nouveau né) à t0-18 h, et à t0, le milieu de culture est remplacé par les chélates vides ou pleins à différentes concentrations (dilution dans le milieu de culture). A t0+72h, les milieux expérimentaux sont remplacés par une solution de XXT, coenzyme Q. Après 3 h d'incubation, on arrête le métabolisme en ajoutant une solution de Sodium Dodécyl Sulfate, SDS. La lecture des D.O est réalisée à 450nm et est proportionnelle à la population cellulaire vivante.

La formule ci-dessous permet de calculer le pourcentage d'activité des chélates :

$$\% \text{ d'activité des chélates} = [(\text{D.O témoin - D.O traité})/\text{D.O témoin}] \times 100$$

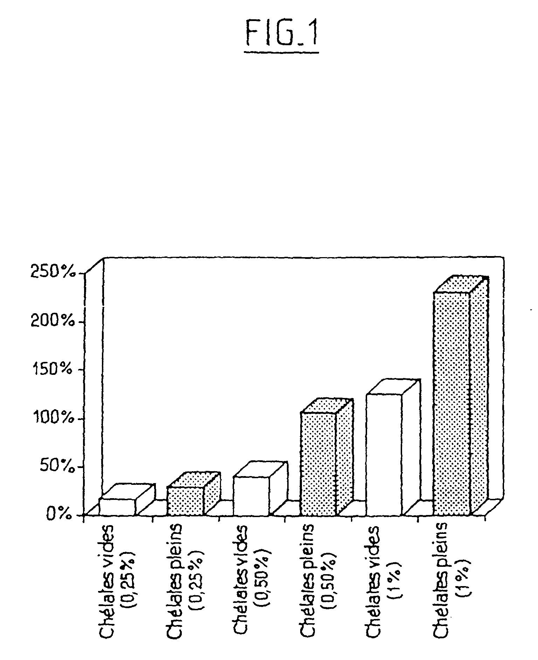

On peut constater d'après les résultats (V. infra, figure 1) que les chélates pleins doublent la prolifération des fibroblastes observée avec les chélates vides.

## Oxygraphie

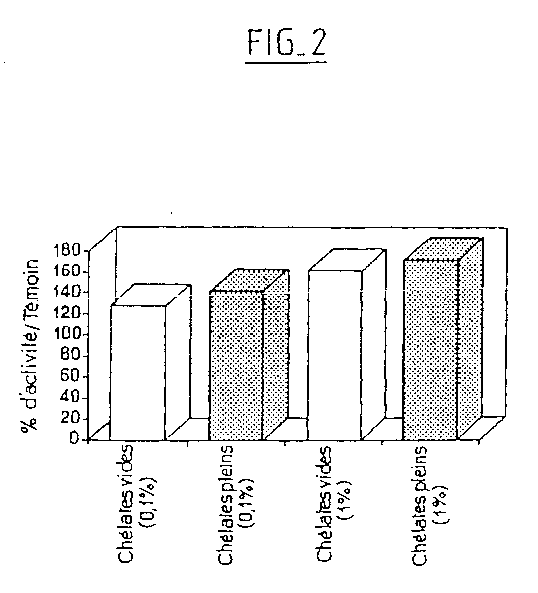

La consommation en oxygène (reflet de la croissance cellulaire), a été mesurée pour des fibroblastes humains en culture avec les chélates pleins, vides, et l'eau thermale seule (V. infra, exemple tableau 2). Les cellules en culture sont incubées pendant 30 minutes avec les chélates vides, pleins et l'eau seule. Le milieu est ensuite prélevé et la concentration en oxygène est dosée. Les résultats (V. infra, figure 2) ont montré une sensible augmentation de la consommation d'oxygène pour les chélates pleins. L'eau seule ne modifie pas la croissance cellulaire.

## Activité anti-radicalaire

L'activité anti-radicalaire des chélates a été évaluée par la technique de Résonance Paramagnétique Electronique (RPE) associée à la technique de piégeage de spin. Le radical hydroxyle a été généré par la réaction de Fenton. Les résultats montrent que les chélates pleins ont une activité deux fois supérieure à celle des chélates vides (Voir tableau 1).

Tableau 1 :

| Activité anti-radicalaire | |
|---|---|
| | concentration d'inhibition 50%, IC50 |
| chélates vides | 0,21% |
| chélates pleins | 0,11% |
| eau thermale seule | 32% |

## Composition de l'eau d'Avène

| ANIONS : | | mg/l | CATIONS : | | mg/l |
|---|---|---|---|---|---|
| | $HCO_3^-$ | 218,4 | | $Ca^{2+}$ | 40,8 |
| | $Cl^-$ | 5,8 | | $Mg^{2+}$ | 22,7 |
| | $SO_4^{2-}$ | 12,4 | | $K^+$ | 1,0 |
| | $NO_3$ | 1,1 | | $Na^+$ | 4 , 8 |
| | $NO_2$ | < 0,02 | | $Li^+$ | < 0,1 |
| | $F^-$ | 0,12 | | $Fe^{2+}$ | < 0,01 |
| | $PO_4^{3-}$ | < 0,1 | | $Mn^{2+}$ | < 0,005 |
| | $Br^-$ | < 0,1 | | $Sr^{2+}$ | 0,13 |

## Composition de l'eau de Cauterets

| ANIONS : | | mg/l | CATIONS : | | mg/l |
|---|---|---|---|---|---|
| | $HCO_3^-$ | 25 | | $Ca^{2+}$ | 5 |
| | $CO_3^{2-}$ | 23,4 | | $Mg^{2+}$ | 0,12 |
| | $H_3SiO_4^-$ | | | $K^+$ | 1,8 |
| | $Cl^-$ | 45 | | $Na^+$ | 63,6 |
| | $SO_4^{2-}$ | 31,5 | | $Li^+$ | 0,18 |
| | $NO_3$ | - | | $NH_4^+$ | |
| | $NO_2$ | - | | $Mn^{2+}$ | |
| | $SO_4^{2-}$ | 31,5 | | $Al^{3+}$ | |
| | $SO_3^{2-}$ | traces | | $Zn^{2+}$ | |

(suite)

| ANIONS : | | mg/l | CATIONS : | | mg/l |
|---|---|---|---|---|---|
| | $S_2O_3^{2-}$ | traces | | $Cu^{2+}$ | |
| | $F^-$ | 2,2 | | | |
| | $PO_4^{3-}$ | - | | | |

Les exemples sont présentés ci-dessous dans un but purement illustratif et ne constituent en aucune espèce une limitation à la présente invention.

**Exemple 1 : Procédé de fabrication des chélates à partir des protéines de blé et de l'eau d'Avène**

1 part de protéines de blé est hydrolysée par voie enzymatique et à pH acide pendant 48h à 50°C. 100 parts d'eau thermale sont concentrées 20 fois jusqu'à l'obtention d'une eau concentrée. Les polypeptides sont séchés et mélangés à l'eau concentrée. Le pH de l'ensemble est neutralisé et une poudre est obtenue par atomisation. La poudre contient 10 % de minéraux de l'eau thermale. Enfin, la poudre est stérilisée par atomisation.

**Exemple 2 : Analyse de la teneur en oligo-éléments dans les chélates**

Les chélates sont précipités dans l'alcool et les oligo-éléments sont quantifiés comparativement à l'analyse de l'eau de départ (Voir tableau 2)

Tableau 2 :

| teneur en oligo-élément dans les chélates | | | |
|---|---|---|---|
| | Eau thermale au griffon | Eau thermale concentrée 20 fois | Chélates pleins |
| Chlorures | 0,0566% | 1,1320% | 11% |
| Sodium | 0,0407g%g | 0,8100% | 8% |
| Sulfates | 0,0223% | 0,4460% | 5% |
| Magnésium | 0,0032g%g | 0,064g%g | 0,51g%g |
| Potassium | 0,00152g%g | 0,0304g%g | 0,39g%g |

Les valeurs obtenues avec l'eau thermale au griffon et les oligo-éléments proviennent des analyses et celles obtenues pour l'eau concentrée proviennent d'un calcul.

On constate que les chélates pleins sont concentrés environ 200 fois dans les chélates pleins comparativement à l'eau de départ.

On obtient in fine les résultats suivants très positifs: A titre d'exemple, 0.5% de magnésium, 0.4% de potassium et 0.01% de strontium (élément très rare),tous étant sous la forme chélatée.

Dans les exemples suivants, les pourcentages sont pondéraux.

**Exemple 3 : Pain dermatologique**

| | Pourcentage |
|---|---|
| Amidon de mais | 25 |
| Lauryl sulfate de potassium | 20 |
| Alcool cetearylique | 20 |
| Cocoyl iséthionate de sodium | 14 |
| Eau déminéralisée qsp | 100 |
| Parfum | 0,3 |
| Chélates pleins | 0,001 à 20% |

**Exemple 4 : Savon**

|  | Pourcentage |
|---|---|
| Suif qsp | 100 |
| Cocoate de sodium | 19 |
| Parfum | 1,5 |
| Cire d'abeille | 0,15 |
| Ester de cétyl | 0,35 |
| Oxydes de fer | 0,02 |
| Dioxydes de titane | 0,02 |
| Chélates pleins | 0,001 à 20% |

**Exemple 5 : Emulsion**

|  | Pourcentage |
|---|---|
| Eau minérale qsp | 100 |
| Huile minérale | 5 |
| Huile de ricin hydrogénée éthoxylée | 5 |
| Triethanolamine | 1,5 |
| Diméthicone | 1 |
| Carbomer | 1 |
| Parfum | 0,2 |
| Chélates pleins | 0,001 à 20% |

**Exemple 6 : Masque**

|  | Pourcentage |
|---|---|
| Eau thermale qsp | 100 |
| Glycérine | 15 |
| Kaolin | 15 |
| Butylène glycol | 7 |
| Hectorite | 4 |
| Glycéyl stéarate | 3 |
| PEG-100 stéarate | 3 |
| Diméthicone | 2 |
| Huile de ricin hydrogénée éthoxylée | 1 |
| Dioxyde de titane | 1 |
| Parfum | 0,1 |
| Triethalonamine | 0,3 |
| Chélates pleins | 0,001 à 20 |

**Exemple 7 : Gelule-voie orale**

|  | Pourcentage |
|---|---|
| Lactose qsp | 100 |
| Chélates pleins | 0,001 à 20 |
| Amidon de blé | 5 |
| Talc | 3 |
| Stéarate de magnésium | 0,5 |

**Exemple 8 : Lait Hydratant**

|  | Pourcentage |
|---|---|
| Eau minérale qsp | 100 |
| Huile de vaseline | 16 |
| Stéarate de polyéthylène glycol | 6 |
| Propylène glycol | 5 |
| Stéarine triple pression | 1 |
| T.E.A | 0,45 |
| Polymère carboxyvinylique | 0,3 |
| 2-Phénoxyéthanol | 0,5 |
| Imidazolidinylurée substituée | 0,15 |
| Parfum | 0,15 |
| Chélates pleins | 0,001 à 20% |

**<u>Références</u>**

- Deby C. et Deby-Dupont G., 1993. Intérêt des chélateurs en cosmétologie pour la prévention du vieillissement cutané par action sur les réactions radicalaires. J. Med. Esth. et Chir. Derm., 19, 111 : 118

- Dreno B., 1995. Oligo-éléments et peau. Cosmétologie n°8-Oct 95

- Leccia M. T., Richard M. J., Favier A, 1993. Intérêts des oligo-éléments en cosmétologie. J. Med. Esth. et Chir. Derm., 20, 175 : 178

- Simonoff M., 1988. Oligo-éléments et thermalisme. Presse Thermale et Climatique, 125 (4)

**Revendications**

1. Composition cosmétique comprenant comme principe actif une combinaison d'oligo-éléments susceptible d'être obtenue par la concentration d'une eau minérale sélectionnée parmi l'eau d'Avène et l'eau de Cauterets, lesdits oligo-éléments étant chélatés par des hydrolysats de protéines végétales.

2. Composition cosmétique selon la revendication 1 **caractérisée en ce que** les chélates proviennent de l'hydrolyse de protéines de blé.

3. Composition cosmétique selon l'une des revendications précédentes **caractérisée en ce que** les peptides chélateurs provenant de l'hydrolyse de protéines ont des poids moléculaires de quelques centaines de dalton.

**4.** Composition cosmétique selon l'une des revendications précédentes **caractérisée en ce qu'**elle comporte d'autres principes actifs en cosmétique et/ou en médecine tels que les rétinoïdes notamment le rétinal, les antiradicalaires dont la vitamine E et les OPC, les antiâges dont l'ADN et les extraits de cyanophycées, les hydratants en particulier l'acide hyaluronique, le glycolate de guanidine et les céramides, les anti-séborrhéiques dont l'extrait de Sabal, les vitamines dont les vitamines C et E, les anti-inflammatoires en particulier l'acide glycyrrhinique, les dépigmentants dont l'extrait d'hydroquinine et l'extrait de piloselle, les antichutes dont le minoxidil, les acides gras essentiels et les huiles riches en AGE, les amincissants, les cicatrisants, les protecteurs solaires, les antifongiques, les antibactériens, les antiviraux, et/ou toute combinaison desdits principes actifs.

**5.** Composition selon l'une des revendications précédentes **caractérisée en ce que** la concentration en chélates est comprise entre 0.0001% et 50% en poids par rapport au poids total de la composition.

**6.** Composition selon l'une des revendications 1 à 5 **caractérisée en ce qu'**elle comporte en outre un ou des véhicules appropriés pour une utilisation en cosmétologie notamment en dermocosmétologie.

**7.** Composition selon l'une des revendications 1 à 5 **caractérisée en ce qu'**elle se présente sous la forme nutraceutique renfermant un ou plusieurs véhicule(s) destiné(s) à permettre son administration par voie orale.

**8.** Utilisation d'une composition selon l'une des revendications 1 à 7 pour améliorer l'état et l'aspect de la peau et/ou des phanères.

**9.** Utilisation d'une composition selon l'une des revendications 1 à 7 pour stimuler la régénérescence et/ou prévenir contre la dégénérescence de la peau.

**10.** Utilisation d'une composition selon l'une des revendications 1 à 7 pour prévenir et lutter contre la formation des radicaux libres et contre leur effet vieillissant sur la peau.

**11.** Utilisation d'une composition selon l'une des revendications 1 à 7 pour lutter contre la sécheresse cutanée.

**12.** Utilisation d'une composition selon la revendication 7 comme produit cosmétique destiné à une administration orale.

**Patentansprüche**

**1.** Kosmetische Zusammensetzung, umfassend als Wirkstoff eine Kombination von Mineral/Spurenelementen, die durch die Konzentration eines Mineralwassers erhältlich ist, das aus Eau d'Avène und Eau de Cauterets ausgewählt ist, wobei die Mineral/Spurenelemente durch Hydrolysate von Pflanzenproteinen chelatisiert sind.

**2.** Kosmetische Zusammensetzung nach Anspruch 1, **dadurch gekennzeichnet, dass** die Chelate aus der Hydrolyse von Weizenproteinen abstammen.

**3.** Kosmetische Zusammensetzung nach einem der vorangehenden Ansprüche, **dadurch gekennzeichnet, dass** die chelatisierenden Peptide, die aus der Hydrolyse von Proteinen abstammen, Molekulargewichte von einigen hundert Dalton aufweisen.

**4.** Kosmetische Zusammensetzung nach einem der vorangehenden Ansprüche, **dadurch gekennzeichnet, dass** sie andere kosmetische und/oder medizinische Wirkstoffe umfasst, wie Retinoide, insbesondere Retinal, Radikalabfänger, darunter Vitamin E und die PCO, Antialterungsmittel, darunter DNA und Extrakte von Cyanophyceae, Feuchtigkeitsspender, insbesondere Hyaluronsäure, Guanidinglycolat und Ceramide, Antiseborrhö-Mittel, darunter Sabal-Extrakt, Vitamine, darunter die Vitamine C und E, entzündungshemmende Mittel, insbesondere Glyzyrrhizinsäure, Depigmentierungsmittel, darunter Hydrochinin-Extrakt und Extrakt von kleinem Habichtskraut, Antihaarausfall-Mittel, darunter Minoxidil, essentielle Fettsäuren und Öle, die reich an EFS sind, schlank machende Mittel, Wundheilungsmittel, Sonnenschutzmittel, Antipilzmittel, antibakterielle Mittel, Antivirusmittel und/oder jede Kombination dieser Wirkstoffe.

**5.** Zusammensetzung nach einem der vorangehenden Ansprüche, **dadurch gekennzeichnet, dass** die Konzentration an Chelaten zwischen 0,0001 und 50 Gew. % einschließlich, bezogen auf das Gesamtgewicht der Zusammensetzung, liegt.

6.  Zusammensetzung nach einem der Ansprüche 1 bis 5, **dadurch gekennzeichnet, dass** sie darüber hinaus ein oder mehrere Vehikel umfasst, die für eine Verwendung in der Kosmetik, insbesondere der Dermokosmetik, geeignet sind.

7.  Zusammensetzung nach einem der Ansprüche 1 bis 5, **dadurch gekennzeichnet, dass** sie in Nahrungsform vorliegt, die ein oder mehrere Vehikel einschließt, das bzw. die dazu bestimmt ist bzw. sind, ihre Verabreichung auf oralem Weg zu ermöglichen.

8.  Verwendung einer Zusammensetzung nach einem der Ansprüche 1 bis 7 für die Verbesserung des Zustandes und des Aussehens der Haut und/oder der Hautanhangsorgane.

9.  Verwendung einer Zusammensetzung nach einem der Ansprüche 1 bis 7, um die Regeneration der Haut zu stimulieren und/oder ihre Degeneration zu verhüten.

10. Verwendung einer Zusammensetzung nach irgendeinem der Ansprüche 1 bis 7, um die Bildung von freien Radikalen und deren Alterungswirkung auf die Haut zu verhüten und zu bekämpfen.

11. Verwendung einer Zusammensetzung nach irgendeinem der Ansprüche 1 bis 7 zur Bekämpfung von Hauttrockenheit.

12. Verwendung einer Zusammensetzung nach Anspruch 7 als kosmetisches Produkt, das für eine orale Verabreichung bestimmt ist.


**Claims**

1.  Cosmetic composition comprising, as active principle, a combination of trace elements which can be obtained by concentrating a mineral water selected from eau d'Avène [Avène water] and eau de Cauterets [Cauterets water], said trace elements being chelated by plant protein hydrolysates.

2.  Cosmetic composition according to Claim 1, **characterized in that** the chelates originate from the hydrolysis of wheat proteins.

3.  Cosmetic composition according to either of the preceding claims, **characterized in that** the chelating peptides originating from the protein hydrolysis have molecular weights of a few hundred daltons.

4.  Cosmetic composition according to one of the preceding claims, **characterized in that** it comprises other active principles in cosmetics and/or in medicine, such as retinoids, in particular retinal, free-radical scavengers, including vitamin E and OPCs, anti-ageing agents, including DNA and extracts of Cyanophyceae, moisturizers, in particular hyaluronic acid, guanidine glycolate and ceramides, anti-seborrhoeic agents, including Sabal extract, vitamins, including vitamins C and E, anti-inflammatories, in particular glycyrrhinic acid, depigmenting agents, including extract of hydroquinine and extract of mouse-ear hawkweed, anti-hairloss agents, including minoxidil, essential fatty acids and EFA-rich oils, slimming agents, cicatrizing agents, sun-screening agents, antifungal agents, antibacterial agents, antiviral agents, and/or any combination of said active principles.

5.  Composition according to one of the preceding claims, **characterized in that** the concentration of chelates is between 0.0001% and 50% by weight relative to the total weight of the composition.

6.  Composition according to one of Claims 1 to 5, **characterized in that** it also comprises one or more vehicles suitable for use in cosmetology, in particular in dermocosmetology.

7.  Composition according to one of Claims 1 to 5, **characterized in that** it is in the nutraceutical form containing one or more vehicle(s) intended to allow its oral administration.

8.  Use of a composition according to one of Claims 1 to 7, for improving the condition and the appearance of the skin and/or of superficial body growths.

9. Use of a composition according to one of Claims 1 to 7, for stimulating regeneration and/or preventing degeneration of the skin.

10. Use of a composition according to one of Claims 1 to 7, for preventing and combating the formation of free radicals, and combating their ageing effect on the skin.

11. Use of a composition according to one of Claims 1 to 7, for combating dryness of the skin.

12. Use of a composition according to Claim 7, as a cosmetic product intended for oral administration.

# FIG.1

# FIG_2